Europäisches Patentamt

European Patent Office

Office européen des brevets

(19)

(11) Publication number: **0 014 300**

**A1**

(12)

# EUROPEAN PATENT APPLICATION

(21) Application number: **79810044.2**

(22) Date of filing: **28.05.79**

(51) Int. Cl.³: **A 61 F 7/00**

(30) Priority: **05.02.79 CH 1077/79**

(43) Date of publication of application:
**20.08.80 Bulletin 80/17**

(84) Designated Contracting States:
**BE DE FR GB IT LU NL SE**

(71) Applicant: **Wath AG**

**9491 Nendeln(LI)**

(72) Inventor: **Bernasconi, Guido**
**Via Ciseri 6**
**CH-6900 Lugano(CH)**

(74) Representative: **Racheli, Adele**
**Racheli & Fiammenghi Via San Gottardo 15**
**CH-6900 Lugano(CH)**

(54) **Independent warming apparatus in particular for neuroarticular pathology therapy.**

(57) The apparatus permitting the use of heat as a therapeutic principle for the neuroarticular pathologies (arthrosis, arthritis, sciatica etc.) comprising an independent source of heat (20) which develops namely heat by catalytic reaction of a fuel and a belt (37) for supporting said source adaptable to that zone of the body which it is desired to treat. A layer of material resistant to heat of well determined thickness and thermal conductivity is interposed between the body and the source of heat (20) to regulate the surface temperature fixed by the therapy.

Fig.5

EP 0 014 300 A1

Independent warming apparatus in particular for neuroarticular pathology therapy.

The present invention refers to an independent warming apparatus in particular suitable for neuroarticular pathology therapy.

In the medical field the use of heat is known as a therapeutic principle in all neuroarticular pathologies such as, for example, arthrosis, arthritis, neuroradicolitis, sciatica and it is also known that the contribution of heat assumes the maximum efficacy when the patient can at the same time keep the painful joint in motion.

The apparatus existing at the present time on the market are constituted by sources of heat which normally are not independent and therefore may be used by the patient only in positions of rest; there are two drawbacks constituted firstly by the lesser efficiency of the supply of heat and secondly by the obvious necessity of reducing the supply time in order not to cause excessive loss of time.

The invention, the subject of the present patent, proposes to present an independent heating apparatus so as to ensure a supply of constant heat for a long period of time at the temperature most adapted to the therapy.

Another object of the invention is that of realizing an

0014300

apparatus which may be applied to the patient rapidly and safely and allow complete freedom of movement of same. A non final scope of the present invention is that of making available an independent heating apparatus which is easily obtainable from elements commonly found in the trade and which in addition is able to give the fullest guarantees of reliability and safety in operation.

The objects mentioned and others which will follow better in the following are obtained by an independent heating apparatus in particular for neuroarticular pathology therapies according to the invention comprising essentially an independent source of heat; a layer of heat resistant material applied to the opposite faces of the source of heat intended to be arranged parallel on the surface of the body of the patient so as to determine the surface temperature fixed for the therapy, the said material being arranged on one of the two faces of clasping means with holding means and keeping in position of the source of heat according to the zone of the body which it is desired to subject to the therapy.

These and other working constructional features of the invention are made evident better from the detailed description which follows having as its object an apparatus according to the invention and illustrated in the attached drawings in which:-

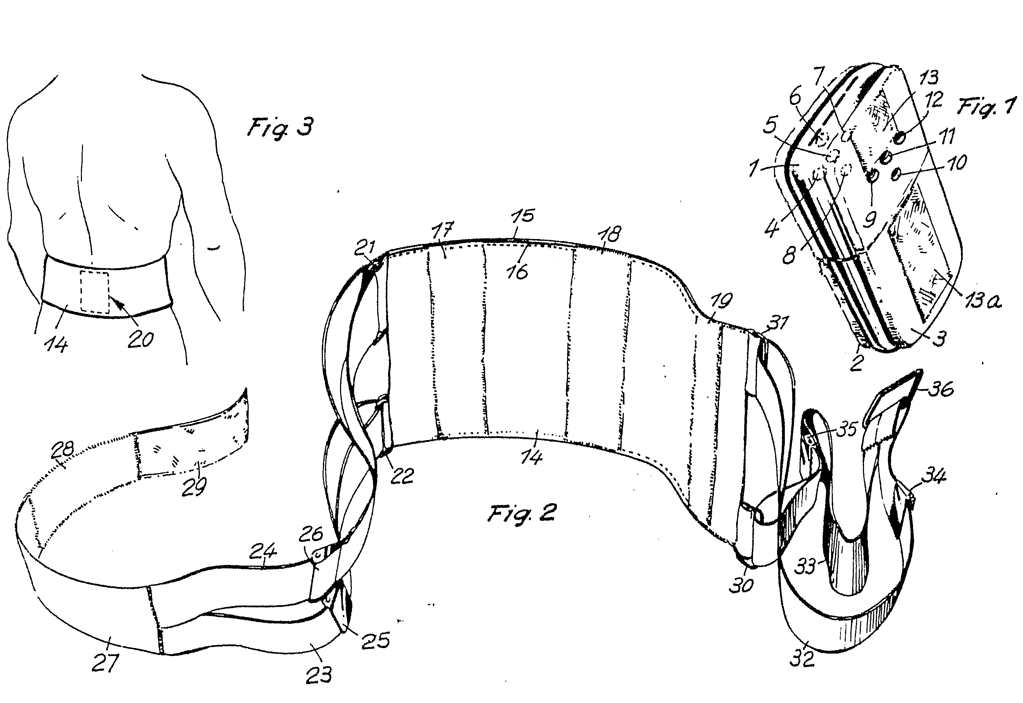

Figure 1 shows the independent source of heat formed by a benzine heater suitable for the pocket covered with two layers of wool felt;

Figure 2 shows the supporting belt of the source of heat in the lumber zone;

Figure 3 shows the application of the apparatus in the lumber zone;

Figure 4 shows the supporting belt of the source of heat of the cervical zone;

Figure 5 shows the application of the apparatus in the cervical zone.

Referring to the above mentioned figures there is indicated with 1 the covering of the independent source of heat of the type, for example, commercially known by the name "Peacock Pocket Warmer" (see USA Patent No. 3,295,510) which contains the reservoir of the benzine and the heat production apparatus by catalytic reaction without flame and on the faces of greater expansion of which are applied by means of glue two layers of material resistant to heat 2 and 3; these layers have the object of reducing the surface temperature from the value $100^{\circ}$C which is verified on the covering 1 to the value $55^{\circ}$C which is kept ideal for the therapy and therefore are constituted by wool felt with a density 0.25 and hardness DIN 61200M4 and have a thickness of three millimeters.

In the zone of the covering provided with vents for the entry of air necessary for the catalytic reduction for the production of heat without flame and for the outlet of the heat developed, there are present on the layer 2 the bores 4,5,6,7,8 and in corresponding positions on the layer 3 the bores 9,10,11,12. These bores allow a hypoalimentation of air to the catalytic reaction apparatus and therefore reduces the temperature reached and prolong the duration of same; from experiments made, it has been found that the said position effects optimize with a diameter of the bores equal to five millimetres.

- 4 -

To the layer of felt 3 is joined the male portion of a pull-off tape formed by the two pieces 13 and 13a.

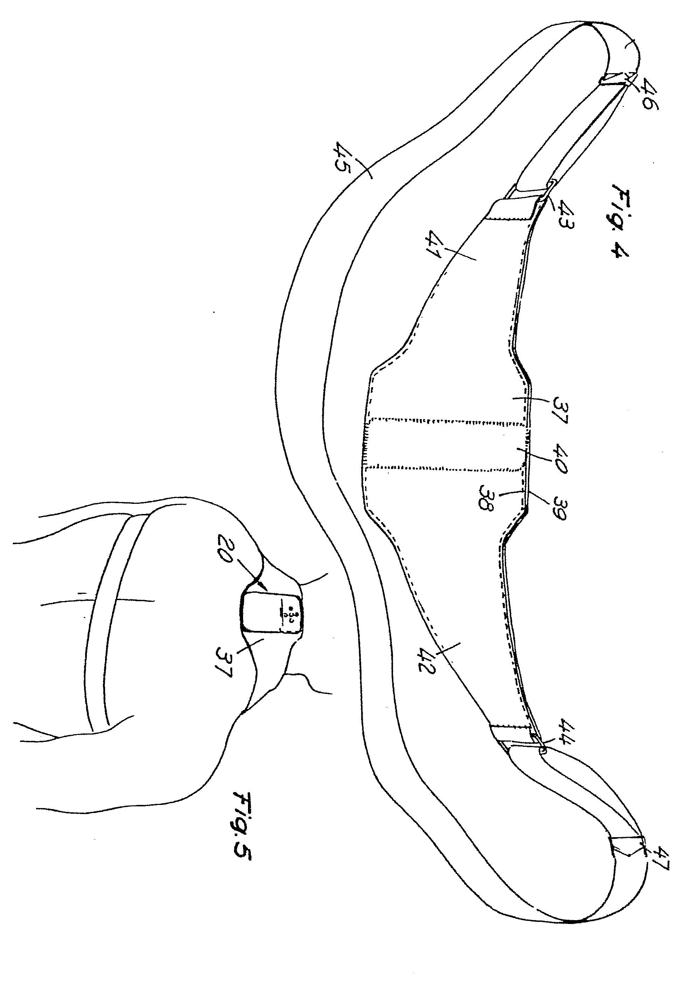

There is indicated with 14 a belt made in two layers 15 and 16 made of starched cotton fabric, for example of the type known commercially under the name "Coutille pesante" to which are joined the female portions 17, 18 and 19 of a tear-off tape to one of which indifferently, according to the therapeutic necessities, may be connected the independent source of heat thanks to the male portion joined to it, for example, in Figure 3 which shows the belt 14 being worn, the source of heat indicated in its entirety with 21 is shown connected to the belt 14 in the central position inserted between the said belt and the body of the patient.

At one end of the belt 14 are connected by means of rings 21 and 22, the elastic tapes 23 and 24 provided with adjusting clasps 25 and 26 and converging to the belt 27 which comprises consecutively the female portion 28 and the male portion 29 of the pull-off tape; at the other end of the belt 14 are similarly connected by means of the rings 30 and 31, the elastic tapes 32 and 33 provided with adjusting clasps 34 and 35 and converging to the rectangular link 36. For the fixing of the belt 14 to the body, after the affixing of the source of heat in the desired position it obviously suffices to insert the belt 27 inside the link 36 supported on the body in the front zone and to cause the closing by the contact of the portions 28 and 29 of the pull-off tape; there is obtained so that a stable contact of the layer 2 of the source of heat with the body of the patient in the desired position.

There is indicated with 37 a belt made similarly to the belt 14 with two layers 38 and 39 made of starched cotton fabric to which is joined in the middle position the female portion 40 of the pull-off tape for the joining with the source of heat and which has two tapering zones 41 and 42 at the end of which is fixed by means of the rings 43 and 44 the elastic tape 45 provided with adjusting clasps 46 and 47. The said belt 37 is adapted to be worn with the methods shown in Figure 5 in order to hold the source of heat 20 applied towards the outside of the belt because in such way the contribution of heat is that sufficient in the cervical zone of the body of the patient. As can be seen the invention satisfies in a way uniformly sound all the requirements made; in particular the fact is emphasized that this allows the patient to keep in contact in the painful zone an independent source of heat at a constant temperature for a period of time which amounts up to eighteen hours so as to cover the entire course of a day with methods so as to allow the greatest liberty of movement; this aspect on the contrary permits of extending the use of the invention to users who do not require specific therapy and desire simply the comfort of a source of heat, for example, during sports training in the interval period.

It is desired to make evident as important also the feature consisting in having realized the supporting belt of the source of heat with a double layer of fabric because it determines a good uniformity of distribution of the heat in the whole lumber or cervical zone.

Claims:

1. An independent heating apparatus in particular for neuroarticular pathology therapy, characterised by comprising an independent source of heat; a layer of material resistant to heat applied to the opposite faces of the source of heat adapted to be arranged parallel to the surface of the body of the patient so as to determine the surface temperature fixed by the therapy, the said material provided on one of the two faces of clasping means with supporting means and holding in position of the source of heat according to the zone of the body which it is desired to subject to therapy.

2. An apparatus according to the preceding claim, characterised in that the source of heat is constituted by a benzine heater suitable for the pocket, formed by a metal casing of substantially parallelepiped shape containing the fuel reservoir and the catalytic reaction for the production of heat without flame, the said casing being provided with vents for communication with the outside for the letting in of the air necessary for the catalytic reaction and for the exit of the generated heat.

3. An apparatus according to the preceding claims, characterised in that the layers of material resistant to heat applied to the faces of the independent source of heat is realized with wool felt of the thickness of three millimeters, density 0.25 and hardness DIN 61200 M4.

4. An apparatus according to the preceding claims, characterised by the presence, on the layer of felt opposite that intended to adhere to the supporting means, of five bores having diameters equal to five millimetres and aligned on the arms of a cross disposed according to the vents of the casing of the source of heat and, on the layer of felt intended to adhere to the supporting means, four bores in position corresponding to that of the bores of the opposite face occupying the three aligned positions along the smaller diameter of the casing of the source of heat and one of the remaining positions.

5. An apparatus according to the preceding claims, characterized in that the clasping means of the independent source of heat to the supporting means of same are constituted by a pull-off tape with the female portion fixed to the said supporting means and the male portion fixed to the source of heat.

6. An apparatus according to the preceding claims, characterised in that the supporting means of the source of heat, according to the lumber zone of the body, are constituted by a belt realized with a double layer of starched cotton fabric, provided with three portions of a female pull-off tape for the fixing of the independent source of heat in various positions, and provided in addition, on the extreme sides, with elastic tapes complete with adjusting clasps and an engaging member so as to allow a fixing of the belt to the body in circumferential positions like a girdle.

7. An apparatus according to the preceding claims, characterised in that the elastic tapes, two in number for each of the extreme sides of the belt, depart from distinct positions on the said extreme sides and the two converge from one part to a rectangular link, and two from the other part, to a belt comprising consecutively a female portion and a male portion of a pull-off tape.

8. An apparatus according to the preceding claims, characterised in that the supporting means of the source of heat according to the cervical zone of the body, are constituted by a belt made with a double layer of starched cotton fabric provided with a female portion of a pull-off tape and suitably fixed to the ends from which a continuous elastic tape derives, comprising adjusting clasps so as to allow a fixing of the belt to the body on a line which, after having gripped the cervical zone goes down the thorax, passes under the arm-pits and terminates on the back.

9.  An independent heating apparatus in particular for neuroarticular pathology therapy according to the preceding claims and as described and illustrated for the purpose specified.

0014300

Fig 3

Fig 2

Fig. 1

2/1

Fig. 4

Fig. 5

| Category | Citation of document with indication where appropriate of relevant passages | Relevant to claim | CLASSIFICATION OF THE APPLICATION (Int Cl3) |
|---|---|---|---|
| X | US - A - 3 159 158 (D.S. BAKER) <br> * entire document * | 1-3 | A 61 F 7/00 |
| | DE - C - 586 682 (O. CHRYSANTH) <br> * claims 1, 2 * | 1,6 | |
| | DE - U - 1 960 377 (ZIMMERMANN & CO.) <br> * claims 1, 11 * | 1 | |
| | DE - U - 1 913 731 (A. KIND) <br> * claims 1, 3; fig. 1 * | 1,6 | **TECHNICAL FIELDS SEARCHED (Int Cl3)** |
| | BE - A - 753 232 (MATSUSHITA ELECTRIC INDUSTRIAL CO.) <br> * claim 1 * | 1 | A 61 F 7/00 |
| | US - A - 3 547 100 (Y. USUI) <br> * claim 1; fig. 3, 9 * | 1 | |
| | US - A - 3 457 908 (M. HAMATANI et al.) <br> * claim 1 * | 1 | **CATEGORY OF CITED DOCUMENTS** |
| A | DE -C - 529 422 (Y. KONO) <br> * fig. 1 * | 1 | X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| A | AT - B - 53 240 (V. FROMMER) <br> * claims 1, 2; fig. 6, 7 * | 1 | |

./..

&: member of the same patent family, corresponding document

| | | |
|---|---|---|
| X | The present search report has been drawn up for all claims | |
| Place of search <br> Berlin | Date of completion of the search <br> 08-05-1980 | Examiner <br> KANAL |

EPO Form 1503.1 06.78

European Patent Office

EUROPEAN SEARCH REPORT

0014300

Application number

EP 79 81 0044

- page 2 -

## DOCUMENTS CONSIDERED TO BE RELEVANT

| Category | Citation of document with indication, where appropriate, of relevant passages | Relevant to claim |
|---|---|---|
| A | FR - A - 821 150 (E. WEHNER) <br> * fig. 1 to 5 * | 1,6 |
| A | US - A - 3 935 856 (W.M. LOFTIN) <br> * claim 1; fig. 1 * | 1 |
| A | US - A - 3 315 658 (J. KAMITANI et al.) <br> * claim 1 * | 1 |
| A | US - A - 2 573 791 (J.N.M. HOWELLS) <br> * claim 1 * | 1 |

CLASSIFICATION OF THE APPLICATION (Int. Cl.³)

TECHNICAL FIELDS SEARCHED (Int. Cl.³)

EP 79 81 0044